(19) **Europäisches Patentamt / European Patent Office / Office européen des brevets**

(11) **EP 3 471 710 B1**

(12) # EUROPEAN PATENT SPECIFICATION

(45) Date of publication and mention
of the grant of the patent:
**11.02.2026 Bulletin 2026/07**

(21) Application number: **17814852.4**

(22) Date of filing: **21.06.2017**

(51) International Patent Classification (IPC):
**A61K 31/122** *(2006.01)*    **A61P 33/14** *(2006.01)*

(52) Cooperative Patent Classification (CPC):
(C-Sets available)
**A61K 9/5161; A01N 25/12; A61K 9/00;
A61K 9/0014; A61K 9/06; A61K 31/12;
A61K 31/122; A61K 36/61; A61K 36/82;
A61K 45/06; A61P 33/14**    (Cont.)

(86) International application number:
**PCT/IB2017/053678**

(87) International publication number:
**WO 2017/221158 (28.12.2017 Gazette 2017/52)**

(54) **ANTI-PARASITIC COMPOSITIONS AND METHODS**

ANTIPARASITÄRE ZUSAMMENSETZUNGEN UND VERFAHREN

COMPOSITIONS ANTIPARASITAIRES ET PROCÉDÉS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.06.2016 AU 2016902431**

(43) Date of publication of application:
**24.04.2019 Bulletin 2019/17**

(73) Proprietor: **University of Canberra
Bruce, Australian Capital Territory 2601 (AU)**

(72) Inventor: **THOMAS, Jackson
Bruce, Australian Capital Territory 2601 (AU)**

(74) Representative: **Mewburn Ellis LLP
Aurora Building
Counterslip
Bristol BS1 6BX (GB)**

(56) References cited:
WO-A1-00/27206            WO-A1-00/42982
WO-A1-02/089587           WO-A1-02/089587
WO-A1-2010/016777         WO-A1-2010/016777
DE-A1- 102007 007 318     DE-A1- 102007 007 318

- **EUN-YOUNG JEONG ET AL: "Acaricidal activity of triketone analogues derived from Leptospermum scoparium oil against house-dust and stored-food mites", PEST MANAGEMENT SCIENCE, vol. 65, no. 3, 1 March 2009 (2009-03-01), BOGNOR REGIS; GB, pages 327 - 331, XP055564698, ISSN: 1526-498X, DOI: 10.1002/ps.1684**
- **"Recent Advances in Novel Drug Carrier Systems", 31 October 2012, INTECH, ISBN: 978-953-51-0810-8, article A. ANTHONY ET AL: "Lipid Nanoparticulate Drug Delivery Systems: A Revolution in Dosage Form Design and Development", pages: 107 - 140, XP055930236, DOI: 10.5772/50486**
- **KHEZRI KHADIJEH ET AL: "Application of nanoparticles in percutaneous delivery of active ingredients in cosmetic preparations", BIOMEDICINE & PHARMACOTHERAPY, ELSEVIER, FR, vol. 106, 24 July 2018 (2018-07-24), pages 1499 - 1505, XP085454923, ISSN: 0753-3322, DOI: 10.1016/ J.BIOPHA.2018.07.084**

- DAYAN, F.E. ET AL.: "Beta-triketone inhibitors of plant p-hydroxyphenylpyruvate dioxygenase: modeling and comparative molecular field analysis of their interactions", J. AGRICULTURAL AND FOOD CHEMISTRY, vol. 57, no. 12, 24 June 2009 (2009-06-24), pages 5194 - 5200, XP055601939
- JEONG, E.Y. ET AL.: "Acaricidal activity of triketone analogues derived from Leptospermum scoparium oil against house-dust and stored-food mites", PEST MANAGEMENT SCIENCE, vol. 65, no. 3, March 2009 (2009-03-01), pages 327 - 331, XP055564698
- DAYAN, F.E. ET AL.: "p-Hydroxyphenylpyruvate dioxygenase is a herbicidal target site for beta-triketones from Leptospermum scoparium", PHYTOCHEMISTRY, vol. 68, no. 14, July 2007 (2007-07-01), pages 2004 - 2014, XP022145453

(52) Cooperative Patent Classification (CPC): (Cont.)

C-Sets
A01N 25/12, A01N 35/06;
A61K 36/61, A61K 2300/00;
A61K 36/82, A61K 2300/00

## Description

### FIELD

The present disclosure relates generally to compositions and formulations useful in the treatment of parasitic infestations of a host subject, including humans, and the host's environment.

### BACKGROUND

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavour to which this specification relates.

Epidermal parasitic skin diseases (EPSD) are a category of infectious skin diseases caused by ectoparasites, whereby the parasite-host interactions are confined to the upper layer of the skin.

Ectoparasites are divided into two main groups, arachnids and insects. The arachnid class includes ticks and mites, and the insect class includes flies, mosquitoes, fleas, and lice. Ectoparasites live on the skin or outgrowths of a host organism, and their biting, chewing or sucking may cause significant detriment to the host. Some ectoparasites are carriers of pathogens which are transmitted while feeding or defaecating; others cause dermatitis and irritations, including raised welts which lead to scratching and potential bacterial infection. Numerous treatments and techniques for their control and eradication are known, including soaps and shampoos, liquids, sprays, dusts and powders, as well as physical removal methods, however, these vary in their level of efficacy and some ectoparasites have developed resistance to the available chemical treatments.

The six major EPSD identified by the World Health Organisation are scabies, pediculosis (capitis, corporis and pubis), tungiasis and hookworm-related cutaneous larva migrans (Bulletin of the World Health Organization; Feb 2009, Vol. 87 Issue 2, p152).

One of the most common ectoparasites of humans is head lice (*Pediculus humanus capitis*). Approximately 25-60% of Australian school children are affected by head lice at any given time (Speare, Richard, and Petra G. Buettner. "Head lice in pupils of a primary school in Australia and implications for control, "International journal of dermatology, 38.4 (1999): 285-290; and https://www2.health.vic.gov.au/public-health/infectious-diseases/disease-information-advice/head-lice).

Untreated infection can lead to poor sleep and excoriation, which can occasionally become super-infected with resistant pathogenic bacteria. Social stigma, embarrassment, low self-esteem, and disgust often plague patients, and many schools prevent children with nits from attending school. Treatments, lost wages and school expenses total an estimated US$4-8 billion (2006 data; Mumcuoglu, K.Y., Meinking, T.A., Burkhart, C.N. and Burkhart, C.G., 2006. Head louse infestations: the "no nit" policy and its consequences. International journal of dermatology, 45(8), pp.891-896).

Conventional synthetic chemical pediculicides may contain neurotoxins and are often associated with allergic and/or toxic effects, and prone to develop resistance. Health departments in Australia recommend wet combing to treat head lice infestation. It is laborious, time consuming and does not produce satisfying cure rates as a treatment on its own, nor does it improve cure rates when used in conjunction with traditional treatments.

Within the past 20 years, head lice have developed resistance to nearly all first-line pharmacotherapy. The worldwide increase in pediculosis is a result of treatment failures resulting from resistance, incorrect application, formulation changes and misdiagnosis, leading to a serious public health problem that is difficult to address. Due to increasing pediculicide resistance in human lice, re-emergence of body louse populations in some geographic areas and demographic groups, persistent head louse infestations, and recent detection of body louse-borne pathogens in head lice and louse-borne diseases are an emerging problem worldwide. The gold standard treatment for head lice is permethrin 1%, for which resistance is extensively documented. Currently available agents have poor ovicidal properties leading to mediocre treatment response and relapse. These difficulties all signal the need to identify new treatments. However, the development of new chemical entities remains doubtful.

Research has shown that no currently available head lice medication is completely effective. The poor efficacy of traditional products and the unproven nature of herbal products are resulting in parental frustration so great that drastic alternatives such as kerosene and veterinary flea products are being used on children in a desperate attempt to cure recalcitrant head lice infestations. However, most government and regulatory authorities are now demanding pest control formulations that are safe, low-residue and that avoid serious environmental contamination from chemical parasiticides.

Scabies is a contagious, parasitic dermatosis caused by the acarine itch mite *Sarcoptes scabiei var. hominis.* It is frequently complicated by bacterial infection leading to the development of skin sores and other more serious consequences such as septicaemia and chronic heart and kidney diseases. In Australia, scabies is a major public health problem in Indigenous communities, affecting 30-65% of schoolchildren (Connors, C. 1994. Scabies treatment. North.

Terr. Commun. Dis. Bull. 2:5-6; and Thomas, J., Peterson, G.M., Walton, S.F., Carson, C.F., Naunton, M. and Baby, K.E., 2015. Scabies: an ancient global disease with a need for new therapies. BMC infectious diseases, 15(1), p.1.).

No current scabicides possess ovicidal, antibacterial, anti-inflammatory and/or anti-pruritic properties to prevent treatment relapse (resulting from newly hatched mites), inflammatory skin reactions (from mite antigens) and pyodermal progression. Most treatments are potentially hazardous and are associated with secondary eczematisation, oedema, erosions and/or pyoderma.

The safety or efficacy of ivermectin (the sole oral therapy) has not been established in the elderly, patients with impaired liver function, children under 5 years of age, and pregnant women. Ivermectin is indicated in Australia only for crusted scabies or cases of typical scabies when prior topical treatment has failed or has been contraindicated. Administration can be labour-intensive since the weight of all patients and the pregnancy status of all women of childbearing age must be determined. Paradoxically, its use is not recommended in those under five years of age nor for households in large communities.

There are reports of resistance of *S. scabiei* to ivermectin *in vitro* and *in vivo,* including treatment failure in clinical trials. Mass drug administration programs have attempted to use ivermectin to control scabies in endemic communities around the world with questionable evidence. Treatment failure of permethrin cream as a scabicide has been documented and it is the slowest acting acaricide (*in vitro*) in the Northern Territory. Permethrin resistance to scabies mites has been confirmed in an animal model and its likely resistance mechanism has also been documented.

Formulations comprising ivermectin to control ectoparasites, including lice, are known, for example, from WO 0042982 A1.

The use of di- and triketones, present in plants, including from the family Leptospermum, are known for their control of pests, including insects, arachnids, molluscs and helminths from WO 02089587 A1.

Long-term adherence difficulties, safety and efficacy uncertainties in the young and elderly, and growing concerns over the development of resistance to classical acaricides (ivermectin and permethrin) all signal the need to identify new treatments for scabies, to reduce the burden of infection in endemic settings and the morbidity and mortality associated with it. It is an object of the present invention to go some way towards meeting this need and/or to provide the public with a useful choice.

There remains a need for alternative treatments of ectoparasitic infestations, in particular epidermal parasitic skin diseases, such as pediculosis and scabies, in humans and animals.

## SUMMARY

It has now been demonstrated that oil obtained from the Manuka tree (*Leptospermum scoparium*), and one or more β-triketones obtainable from the oil of the Manuka tree, leptospermone, isoleptospermone, grandiflorone and flavesone, have anti-ectoparasictical activity. Compositions and formulations comprising one or more β-triketones may provide an effective treatment for ectoparasitic infestation of an animal host and/or its environment. The compositions and formulations may be useful in the control of epidermal parasitic skin diseases, in humans and animals, such as pediculosis and scabies.

In a first aspect, there is provided an ectoparasite controlling nanoparticle composition comprising one or more β-triketones selected from leptospermone, isoleptospermone, grandiflorone and flavesone, together with a carrier, wherein the carrier is chitosan or comprises chitosan, and wherein the one or more β-triketones is a botanical extract obtained from *Leptospermum scoparium.*

In another aspect, there is provided a nanoparticle composition as in the first aspect for use in the treatment of an ectoparasitic infestation of a host subject comprising the topical administration of the said nanoparticle composition.

In another aspect, there is provided a non-therapeutic method for controlling an ectoparasitic infestation in a host's environment, comprising the step of applying to said environment a nanoparticle composition according to the first aspect.

In one aspect, there is provided a non-therapeutic method of killing the eggs of an ectoparasite, the method comprising contacting the eggs with the nanoparticle compositions according to the first aspect.

With respect to non-therapeutic methods, in some embodiments, the ectoparasite is selected from the group comprising head lice, body lice or pubic lice. In another embodiment the ectoparasite is selected from a mite or tick.

The nanoparticle composition comprises one or more β-triketones, wherein the one or more β-triketones is a botanical extract obtained from *Leptospermum scoparium.* In some embodiments, the nanoparticle composition comprises at least leptospermone. In some embodiments, the nanoparticle composition comprises at least isoleptospermone. In some embodiments the nanoparticle composition comprises at least flavesone. In some embodiments, the nanoparticle composition comprises at least grandiflorone. In some embodiments, the nanoparticle composition comprises at least two or all three of leptospermone, isoleptospermone, grandiflorone and flavesone. In some further embodiments, the nanoparticle composition comprises a mixture of leptospermone, isoleptospermone, grandiflorone and flavesone, obtainable from a botanical source, such as *Leptospermum scoparium.*

The pharmaceutically acceptable carrier is or comprises a polymeric polysaccharide carrier. The polysaccharide carrier is chitosan, or comprises chitosan.

The botanical extract obtained from *Leptospermum scoparium* comprises at least one β-triketone selected from leptospermone, grandiflorone, isoleptospermone and flavesone. In further embodiments, the botanical extract comprises at least two, three or all four of leptospermone, isoleptospermone, grandiflorone and flavesone. In some further embodiments, the botanical extract is at least about 90% pure, that is to say comprises at least about 90%, or at least about 95% or at least about 99% one or more β-triketone(s). The botanical extract may be formulated with a suitable carrier and/or one or more acceptable additives.

In some embodiments, the ectoparasite is selected from lice, fleas, mites and ticks. In further embodiments, the compositions, formulations, methods and uses are for treating or controlling epidermal parasitic skin diseases such as pediculosis or scabies in humans or animals.

## BRIEF DESCRIPTION OF THE FIGURES

**Figure 1** depicts chromatogram plots of Fractions 1-3 obtained by distillation of Manuka oil (2nd -4th chromatogram plots from the top, respectively) and of neat Manuka oil (top chromatogram). Fraction 3 is enriched in the β-triketones, leptospermone, isoleptospermone, grandiflorone and flavesone in the following proportions: leptospermone (68.6%), isoleptospermone (15.5%), grandiflorone (0.7%) and flavesone (15.2%);

**Figure 2** provides a pictorial summary of a method of making chitosan nanoparticles;

**Figure 3** graphically depicts the pediculicidal activity for Manuka oil (5% neat in soy bean oil) and Manuka oil nanoparticle formulation (5%) against *P. humanis,* compared to conditioner, permethrin and tea tree oil;

**Figure 4** graphically depicts the ovicidal efficacy of 2% and 5% Manuka oil solutions and a 5% Manuka oil nanoparticle formulation against *P. humanis* nits, compared to permethrin and ivermectin;

**Figures 5A-D** graphically depict the survival rates of scabies mites when treated with 10% neat Manuka oil solution, tea tree oil solution, Manuka oil fractions 1-3, incorporated into Emulsifying ointment (BP88). Manuka oil was fractionated by vacuum distillation under reduced pressure (20-25 mm) with gentle heating. Fractions were collected at regular intervals (over defined boiling-point ranges) (fraction-1 was collected at 35-40°C and approximately 20 mm pressure; fraction- 2 at 40-45°C , ~25 mm pressure; fraction 3 at 45-52.5°C, ~25 mm pressure);

**Figure 6** graphically depicts the survival time of scabies mites for 5% Manuka oil nanoparticle formulation compared to permethrin and ivermectin; and

**Figure 7** shows the survival rates of sheep body lice (*Bovicola ovis*) when treated with 5% neat Manuka oil, and 1%, 2% and 3% Manuka oil nanoparticle formulations compared to ivermectin and negative controls.

## DESCRIPTION

Throughout this specification and the claims which follow, unless the context requires otherwise, the word "comprise" and variations such as "comprises" and "comprising" will be understood to imply the inclusion of a stated integer or step or group of integers but not the exclusion of any other integer or step or group of integers or steps.

Throughout this specification and the claims which follow, unless the context requires otherwise, the phrase "consisting essentially of", and variations such as "consists essentially of" will be understood to indicate that the recited element(s) is/are essential i.e. necessary elements of the invention. The phrase allows for the presence of other non-recited elements which do not materially affect the characteristics of the invention but excludes additional unspecified elements which would affect the basic and novel characteristics of the invention defined.

The singular forms "a", "an" and "the" include plural aspects unless the context clearly dictates otherwise.

The term "invention" includes all aspects, embodiments and examples as described herein.

As used herein, "about" refers to a quantity, value or parameter that may vary by as much as 25%, 20%, 15%, 10%, 5%, or 1-2%, and includes at least tolerances accepted within the art.

The botanical extracts, compositions and formulations of the disclosure comprise at least one, or at least two or at least three or all four β-triketones selected from leptospermone, grandiflorone, isoleptospermone and flavesone.

The concept of a "natural" treatment has great appeal to some consumers, particularly where alternative available treatments rely on the use of synthetic agents. Thus, the β-triketone is obtained from a botanic source. β-Triketones are found mainly in the trees and shrubs of *Myrtaceae* family, particularly in certain species of the genera *Leptospermum, Callistemon,* and *Eucalytpus.* One such exemplary species is, the Manuka tree (*Leptospermum scoparium*), a species of flowering plant native to New Zealand and to southeast Australia. Leptospermone, isoleptospermone, grandiflorone and flavesone are components of manuka oil, obtained from the bark and leaves of *Leptospermum scoparium.* Some other species of the *Myrtaceae* family, such as Lemon Bottlebrush (*Callistemon citrinus*), a shrub native to Australia, and other *Leptospermum* species, as well as *Eucalyptyus* species are other sources of the β-triketones.

When obtained from a botanic source, the β-triketone may be present in an extract, such as an oil, obtained from any one or more of bark, stems, leaves, roots, seeds, flowers *etc.* As used herein, "botanical extract" includes the mixture of components extracted directly from the botanical source, as well as further fractionated and/or isolated or purified forms. In some examples, an initial botanical extract may be obtained by steam distillation or solvent extraction of the botanic material, optionally ground, crushed or otherwise comminuted. In further examples the further fractionated and/or isolated or purified forms may be obtained by any suitable means, for example, fractional distillation and/ or chromatography. Depending on the botanic source and the extraction and any further fractionation and/or purification processes employed, the extract may contain one or more other botanical components derived from the botanical source, which may be a β-triketone other than leptospermone, isoleptospermone, grandiflorone and flavesone, or any other component. In other embodiments, the extract is further fractionated to afford one or more β-triketones in purified form, for example, having at least about 90% purity or at least about 95% purity, or at least about 97-98% purity, or even at least about 99%, or 99.5%, purity. Where there is a mixture of two or more of the β-triketones, reference to purity refers to the mixture of β-triketones selected from leptospermone, isoleptospermone, grandiflorone and flavesone. For example at least 95% purity denotes that 5% or less is a component or components other than the above-mentioned four β-triketone(s). Purity may be determined by methods known in the art, for example mass spectrometry, nuclear magnetic resonance techniques and chromatography, such as HPLC and GC/MS. The botanical extract, including a fraction thereof, or isolated or purified β-triketone(s), may be further formulated with a suitable carrier and one or more suitable additives.

It will be understood that whilst the one or more β-triketone component(s) according to the invention is obtained from botanical sources, the β-triketone(s) can also be chemically synthesized. For example, leptospermone can be synthesized from phloroglucinol by a reaction with 3-methylbutanenitrile (isovaleronitrile) in the presence of a zinc chloride catalyst to form the acyl phloroglucinol. Alkylation with iodomethane after initial treatment with sodium ethoxide and ethanol produces an intermediate which is treated with aqueous hydrochloric acid to afford leptospermone. Analogous procedures, using the appropriate nitrile, can be used to prepare isoleptospermone, flavanone and grandiflone. See also Van Klink, J.W., et al, in J. Nat. Prod., 1999, 62(3), pp 487-489 and Briggs, L. H., et al, J Chem. Soc.174, 383, 1948.

β-Triketone components, either as extracts containing other botanical components, or purified forms thereof, may also be purchased from commercial suppliers. Individual triketone components that have been purified from botanical sources, synthesized or purchased may be combined, mixed or blended to provide a combination or mixture of β-triketone components, either approximating or mimicking that found in a botanic source, or to enrich one or more components compared to the botanic source. Synthesized β- triketone(s) may be used in purified form, as described above.

It will be understood that the β-triketones referred to herein are capable of existing in keto-enol tautomeric forms, as well as racemic and individual stereoisomeric forms and that all such forms are contemplated herein.

The nanoparticle compositions comprise leptospermone or grandiflorone, or isoleptospermone, or flavesone, as a botanical extract obtained from *Leptospermum scoparium.*

In some embodiments the nanoparticle composition comprises leptospermone and isoleptospermone. In some embodiments the nanoparticle composition comprises leptospermone and flavesone. In some embodiments the nanoparticle composition comprises leptospermone and grandiflorone. In some embodiments the nanoparticle composition comprises isoleptospermone and flavesone. In some embodiments the nanoparticle composition comprises isoleptospermone and grandiflorone. In some embodiments the nanoparticle composition comprises flavesone and grandiflorone.

In some embodiments, the nanoparticle composition comprises leptospermone, isoleptospermone and flavesone. In some embodiments, the nanoparticle composition comprises leptospermone, isoleptospermone and grandiflone. In some embodiments, the nanoparticle composition comprises leptospermone, flavesone and grandiflone. In some embodiments, the nanoparticle composition comprises isoleptospermone, flavesone and grandiflone.

In still further embodiments, the nanoparticle composition comprises leptospermone, isoleptospermone, flavesone and grandiflorone.

In still further embodiments, where the nanoparticle composition comprises at least leptospermone, it is present as the major β-triketone consitutuent, that is to say, the amount of leptospermone is greater than the amount of any other single β-triketone component. In some embodiments, the amount of leptospermone is greater than the total amount of all other β-triketone components.

Two or more β-triketones, when used in conjunction, either in a single composition or formulation or discrete compositions or formulations may have a synergistic or additive anti-ectoparastical effect.

Nanoparticle carriers of the disclosure are or comprise chitosan.

The carrier for the nanoparticles consists of or comprises the polysaccharide, chitosan.

Polysaccharides are polymers of repeating monosaccharide units linked by glycosidic bonds and are readily found in nature from a variety of sources, including algae, plants, microbes and animals. Depending on the nature of the monosaccharide repeat units, they may be classed as homopolysaccharides or heteropolysaccharides, and may be positively charged, negatively charged or neutral depending on the pH of their environment.

The polysaccharide carrier may be further chemically modified and/or may contain other non-polysaccharide components such as glutaraldehyde, polyethylene glycol, acrylic acid, polyacrylic acid, poly-γ-glutamic acid, and methacrylic acid. Common methodologies for the preparation of polysaccharide nanoparticles include covalent cross-linking, ionic crosslinking, polyelectrolyte complexation and self-assembly of hydrophobically modified polysaccharides [Lui, Z., et al, Polysaccharides-based nanoparticles as drug delivery systems, Adv Drug Deliv Rev, 60(15), 1650-1662, 2008].

The polysaccharide is chitosan, or in some exemplary embodiments, a combination of chitosan and at least one other polysaccharide, such as alginate.

Varied formulation methods may be employed to prepare chitosan nanoparticles, including but not limited to emulsification and crosslinking, different types of coacervation, or even slight modifications of these. Methods may include emulsion-droplet coalescence (Tokumitsu H, et al. 1999. Chem Pharm Bull, 47, 838-842), emulsion solvent diffusion (El-Shabouri MH. 2002. Int J Pharm, 249, 101-108), reverse micellar method (Mitra S, et al. J Control Rel, 74, 317-323), ionic gelation, polyelectrolyte complexation (Calvo P, et al. 1997. J Appl Polym Sci, 63, 125-132., Sarmento B, et al, 2006a. Int J Peptide Res Ther, 12, 131-138) and desolvation (Tian XX, Groves MJ. 199, J Pharm Pharmacol, 51, 151- 157). A partial summary of different methods applied to produce chitosan-based nanocarriers, as well as their matrix composition is presented below in Table A.

**Table A.** Some key methods used for the production of chitosan-based nanoparticles and composition of the carriers' matrix

| Production Method | Matrix composition | Key References |
|---|---|---|
| Emulsification and cross-linking | Chitosan, glutaraldehyde | Ohya et al. 1994; Songjiang Z, Lixiang W. 2009, AAPS Pharmsci-Tech, 10, 900-905 |
| Emulsion droplet coalescence | Chitosan | Tokumitsu et al. 1999; Anto SM, et al. 2011. Int J Pharm Biol Arch, 2, 926-931 |
| Emulsion solvent diffusion | Chitosan | El-Shabouri 2002 |
| Reverse micellisation | Chitosan, glutaraldehyde | Mitra et al. 2001; Manchanda R, Nimesh R. 2010. J Appl Polym Sci, 118, 2071-2077 |
| Ionic gelation | Chitosan, sodium tripolyphosphate | Calvo et al. 1997, Fan W, et al. 2012. Colloid Surf B-Biointerfaces, 90, 21-27 |
| Polyelectrolyte complexation | Chitosan, alginate, arabic gum, carboxymethyl cellulose, carrageenan, chondroitin sulfate, cyclodextrins, dextran sulfate, polyacrylic acid, poly-γ-glutamic acid | Kaihara S, et al. 2011. Colloid Surf B - Biointerfaces, 85, 343- 348; Yeh MK et al 2011. Acta Biomater, doi:10.1016/j.actbio.2011.06.026. |
| Modified ionic gelation with radical polymerisation | Chitosan, acrylic acid, methacrylic acid, polyethylene glycol, polyether | Hu Y, et al. 2002. Biomaterials, 23, 3193-3201; Sajeesh S, Sharma CP. 2006a. Int J Pharm, 325, 147-154. |
| Desolvation | Chitosan | Agnihotri SA, Aminabhavi TM. 2007. Drug Develop Ind Pharm, 33, 1254-1262; Atyabi F, et al. 2009. J Nanosci Nanotechnol, 9, 4593-4603 |

Adopted from Grenha A. 2012. Chitosan nanoparticles: a survey of preparation methods. Journal of drug targeting; 20:291-300.

The nanoparticle compositions of the disclosure may be prepared by any suitable process. An exemplary process may include one or more steps of:

- preparing an aqueous solution the carrier comprising chitosan to provide an aqueous phase;
- adding to said aqueous phase an oil phase comprising a solution of the one or more β triketone(s), or botanical extract, such as Manuka oil, in a suitable oil carrier;
- homogenizing the aqueous and oil phases to obtain an oil-in-water emulsion;
- forming nanoparticles by any suitable means, such as ultrasonication;
- freeze-drying the particles.

The nanoparticles of the disclosure have a diameter of about 1000 nm or less, such as about 500 nm or less, and typically in the range of about 50 - 350 nm. In some further embodiments, the nanoparticles have a diameter of about 60 to about 80 nm, or about 70 to about 90 nm, or about 80 to about 100 nm, or about 90 to about 110 nm or about 100 to about 120 nm or about 110 to about 130 nm. In other embodiments, the nanoparticles have a diameter of about 70 to about 130 nm, such as about 70 to about 100 nm. In other embodiments, the nanoparticles have a diameter in the range of about 80-350 nm, such as in the range of about 100-300nm, or in the range of about 200-250 nm. The size of the nanoparticles may vary depending on, for example, the formulation procedures employed and concentration of active ingredient loaded, type of carrier used (e.g. *N*, *N*-Dimethyl chitosan vs. *N, N, N*-trimethyl chitosan), as well as the age of the formulation. Particle size may be measured by any suitable means, such as scanning electron microscope (SEM), atomic force microscope (AFM) or transmission electron microscope (TEM). In some examples, the nanoparticles, such as where the polysaccharide is or comprises chitosan, have a diameter in the range of about 80-350, such as about 90 to about 130 nm as measured by SEM. In some embodiments, the mean diameter, immediately after preparation may be about 90 $\pm$5 nm.

As used herein, the term "ectoparasite" is taken to include any parasitic species that lives externally on a host subject, reproducing by laying eggs. Examples of ectoparasites are found in the class of *Arachnida* (*e.g.* mites, bedbugs and ticks) or *Insecta* (*e.g.* fleas and lice). Further non-limiting examples include species from the orders *Phthiraptera, Siphonaptera, Hemiptera, Acariformes,* (including *Parasitiformes and Sarcoptiformes).*

In some embodiments of the disclosure the ectoparasite is a louse, (plural: lice) the common name for members of the order *Phthiraptera.* Lice live externally on warm-blooded hosts which include most species of bird and mammal. Chewing lice (suborder *Mallophaga*) live among the hairs or feathers of their host and feed on skin and debris, while sucking lice (suborder *Anoplura*) pierce the host's skin and feed on blood and other secretions.

Humans host three species of louse, the head louse (*Pediculus humanus capitis*)*,* the body louse (*Pediculus humanus humanus,* sometimes called *Pediculus humanus corporis*) and the pubic louse, or crab louse (*Phthirus pubis*). Head lice spend their entire life on the human scalp feeding exclusively on blood, attaching their eggs to scalp hair rather than to clothing. Body lice attach their eggs to clothes. The eggs of the crab louse are laid usually on the coarse hairs of the genital and perianal regions of the human body. Crab lice may also be found on other areas of the body that have coarse and relatively sparse coverings of hair, such as the beard, moustache, eyelashes, underneath the arms. They do not generally occur on the finer hair of the scalp. The condition of being infested with lice, such as head lice, body lice, or pubic lice is known as pediculosis.

Thus in some embodiments, the ectoparasite is selected from head lice, body lice or pubic lice. In further embodiments, there is provided a nanoparticulate composition for use in treating a lice infestation (pediculosis) in a human or animal host, comprising the topical administration of a nanoparticle composition of the disclosure to said host and/or said host's environment. Also contemplated are formulations and agents for treating a lice infestation (pediculosis) in an animal or human host.

In other embodiments, the ectoparasite is a mite or a tick. Mites, along with ticks, are small arthropods belonging to the subclass *Acari* and the class *Arachnida.*

Scabies is caused by infection with the female mite *Sarcoptes scabiei.* The mites burrow into the skin to live and deposit eggs. The symptoms of scabies are due to an allergic reaction to the mites. Scabies is most often spread during a relatively long period of direct skin contact with an infected person. Scabies may also occur in a number of domestic and wild animals; the mites that cause these infestations are of different subspecies from the one typically causing the human form. The most frequently diagnosed form of scabies in domestic animals is sarcoptic mange, caused by the subspecies *Sarcoptes scabiei canid,* and occurs most commonly in dogs and cats. Thus, in still further embodiments of the disclosure, the ectoparasite is *Sarcoptes scabiei.* In further embodiments, there is provided a nanoparticle composition for treating scabies in a human host, comprising the topical administration of a nanoparticle composition of the disclosure to said host. Also contemplated are formulations and agents for treating scabies in an animal or human host.

Dust mites feed on organic detritus, such as flakes of shed human skin, and live in indoor environments such as furniture, bedding and carpets. House dust mites are a common cause of asthma and allergic symptoms. Thus, in still other embodiments, the ectoparasite is a dust mite (*Dermatophagoides pteronyssinus* and *Dermatophagoides farinae).* The nanoparticle compositions of the disclosure may be sprayed or dusted on furniture, bedding (including mattresses, mattress protectors, blankets, pillows and cushions), curtains and carpets and rugs.

In some embodiments, the ectoparasite is a flea. Fleas are blood sucking insects that form the order *Siphonaptera.* Flea bites cause the skin to raise, swell, and itch, and it is the itching that leads to scratching in the vicinity of the bite. Fleas can spread rapidly and move between areas to include eyebrows, eyelashes, and pubic regions. Hair loss as a result of itching is common, especially in wild and domestic animals. Anemia is also possible in extreme cases of high-volume infestations. Thus, in some embodiments the ectoparasite is a human flea (*Pulex irritans*), a dog flea (*Ctenocephalides canis*) or a cat flea (*Ctenocephalides felis*).

Other ectoparasites contemplated herein may include bed bugs (such as the common bed bug, *Cimex lectularius,* and the tropical bed bug, *Cimex hemipterus*).

The nanoparticle compositions of the inventions may be useful in treating one or more of the breeding cycle stages, including the egg, nymph and adult stages of the ectoparasite. Whilst many treatments have some efficacy against the adult or nymph stages, the eggs often remain; and unless the eggs are also prevented from hatching, reinfestation with nymphs and adults can occur. The compositions and formulations of the disclosure may also have useful ovicidal activity and inhibit, disrupt or otherwise prevent the hatching of ectoparasitic eggs, characterised by the opening of the operculum of the egg and emergence of the nymph. Accordingly, in some advantageous embodiments some compositions and formulations of the disclosure may be effective in both killing adult and/or nymph ectoparasites, as well as killing, or preventing the egg from hatching. Thus, another aspect of the disclosure provides an ovicidal nanoparticle composition comprising one or more β-triketones selected from leptospermone, isoleptospermone, grandiflorone and flavesone, together with a chitosan

carrier. The one or more β-triketones is a botanical extract, obtained from the Manuka tree (*Leptospermum scoparium*). Yet a further aspect provides an ovicidal composition comprising a botanical extract comprising one or more β-triketones selected from leptospermone, grandiflorone, isoleptospermone and flavesone. The botanical extract may be Manuka oil or a fraction thereof.

Host subjects to be treated may be human or non-human, and include: mammalian subjects such as humans, primates, livestock and herd animals (including cows, horses, sheep, pigs, deer, buffalo, alpacas, llamas and goats,), companion animals (including dogs, cats, rabbits, guinea pigs, hamsters, rats, mice), and other domesticated animals, as well as domesticated and captive wild animals. Laboratory animals such as rats, mice, rabbits, hamsters, and primates may also be treated in accordance with the disclosure. Non-mammalian species such as birds (e.g., chickens, geese, turkeys, ducks, guinea fowl, parrots, pigeons), amphibians and fish may also be contemplated in certain embodiments of the disclosure.

The botanical extracts compositions and formulations of the disclosure are administered or applied in a treatment or controlling effective amount. A treatment or controlling effective amount is intended to include an amount which, when administered or applied according to the desired dosing regimen, treats or controls the ectoparasitic infestation, that is to say, at least partially attains the desired effect of reducing the extent of, preventing or slowing the population growth of, or eradicating an ectoparasitic infestation, and includes one or more of killing the adult or nymph stage of the ectoparasite, preventing the adult from reproducing, interrupting the progression of a nymph moulting stage, killing eggs or otherwise preventing the eggs from hatching.

Suitable dosage amounts and dosing regimens may depend on the particular ectoparasitic infestation being treated, whether the infestation is on a host subject or has extended to its surroundings, the severity of the infestation, as well as the general age, health and weight of the subject. Dosages may be administered once, or multiple (*e.g.* 2, 3 or 4) times daily, or one or more times weekly (*e.g.* 2, or 3 times a week, such as every second or third day), fortnightly or monthly. The botanical extracts, compositions and formulations of the disclosure may be administered according to the desired dosing regimen until the desired result is achieved, e.g. the infestation is eliminated or eradicated, reduced, or that the breeding cycle of the ectoparasite is disrupted. In advantageous embodiments, the botanical extracts, compositions and formulations of the disclosure may be used on the host or its environment and surroundings as soon as the presence of an ectoparasite is identified, or if the potential for an infestation arises, that is to say, in a preventative sense. Thus, the disclosure also relates to a method of preventing an ectoparasitic infestation of a host subject or its environment by applying an extract, composition or formulation of the disclosure to said host or environment.

The β-triketones, botanical extracts and nanoparticle compositions of the disclosure may be used directly, but are preferably prepared as a formulation together with one or more suitable carriers and/or additives. Some exemplary formulations, suitable for topical application to a host subject, and/or its environment, include: gels, shampoos, powders, dusts granules, ointments, creams, lotions, washes, liquids, solutions, pour-ons, rinses and sprays (aerosol and non-aerosol). Some non-limiting examples of carriers suitable for pharmaceutical or veterinary use may include water alcohols (e.g. ethanol and isopropanol), hydrocarbons, methylcellulose, hydroxymethylcellulose, dextran, alginate, pectin, guar, xanthan gum, gum Arabic, carrageenan, aloe vera, Hoheria, dehydroxantahan gum (e.g .Amaze™ XT), Dermofeel, Tween 20, Carbopol, oils, such as olive oil, soy bean oil, vegetable oil, castor oil *etc,* and mixtures thereof. In addition to carriers, the formulation may optionally also contain one or more additives suitable for veterinary use, such as stabilizers, emulsifiers, binders, dispersants, surfactants, buffers, suspending agents, gelling agents and viscosity modifiers, perfumes, and preservatives. Processes for the preparation of such formulations are known to those skilled in the art and, together with exemplary carriers and additives, are described, for example in Remington: The Science and Practice of Pharmacy, 22nd Edition, 2012. Compositions and formulations may also be coated or painted onto, or impregnated or incorporated into devices to be worn by or attached to the host, for example, ear tags and collars.

The formulations and compositions may contain one or more β-triketones, for example a single β-triketone, or a mixture of 2, 3 or 4, in an amount of from 0.001% to about 50% by weight or volume of the formulation or composition, such as from about 0.001% to about 25%, or about 0.01 to about 20%, or about 0.05 to about 15%, or 0.5% to about 10%, or about 1% to about 5% or about 3 to about 8%

The extracts, compositions and formulations of the disclosure are useful in the treatment or control of ectoparasite infestations of human and non-human host subjects, and their surroundings or environments, particularly in the treatment or control of EPSD such as pediculosis or scabies. The extracts, compositions and formulations are typically applied to the external areas of the host where the infestation occurs, such as skin, hair, coat or fur, such that the composition or formulation comes into direct contact with the ectoparasite, for example by spraying, dusting, rubbing, coating, spreading, combing through or shampooing. For example, treatment of an infestation of head lice in a human can entail applying the composition or formulation to the head and hair as a spray, or gel or shampoo. Treatment of scabies can involve applying the composition or formulation as a spray, ointment, cream, lotion or gel directly to the skin.

Whilst an ectoparasite may live on a host subject, for example, skin, hair, coat, fur or fleece, or within or proximate to body crevices and openings, they may in certain circumstances also be found in the host subject's environment, such as clothing, bedding (e.g. mattresses, pillows, blankets, quilts sheets, cushions and duvets), carpets and rugs, window furnishings such as curtains and blinds, other soft furnishings, including cushions, pillows and blankets; as well as floorboards, paintings, behind skirting and architraves, in cracks and crevices of walls, within bed frames and other furniture, and behind loose wallpaper. In the case of animal host environments, ectoparasites may also be found in protective coverings (such as coats), bedding, including blankets, mattresses and pillows; straw, hay; wood shavings *etc*; and shelter constructions (such as barns, sheds, stalls, hutches, coops, cages and aviaries). Thus, another aspect of the disclosure provides, a method for controlling or eradicating an ectoparasitic infestation in a host's environment, comprising the step of applying a compositions and formulations of the disclosure to said environment. The compositions and formulations may be applied by any suitable means including dusting, spraying, sprinkling, painting, washing, spreading, rubbing into, or pouring on to the environment and its proximity.

## EXAMPLES

Manuka oil (*Lepstospermum scoparium* oil) was obtained from Phytomed, Auckland, New Zealand.

### *Manuka Oil Fractionation*

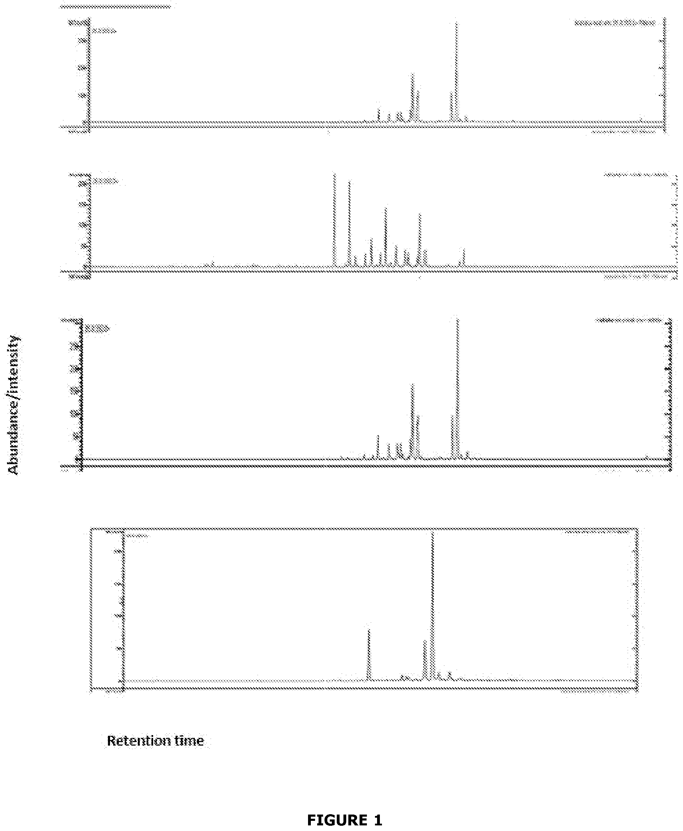

Manuka oil was fractionated by vacuum distillation under reduced pressure (20-25 mm) with gentle heating. Essential oil fractions were separated based on the boiling points of their terpene constituents. Fractions were collected at regular intervals (over defined boiling-point ranges) (fraction-1 was collected at 35-40°C and approximately 20 mm pressure; fraction- 2 at 40-45°C , ~25 mm pressure; fraction 3 at 45-52.5°C, ~25 mm pressure) and refined further by column chromatographic technique [Porter, Noel G., and Alistair L. Wilkins, Chemical, physical and antimicrobial properties of essential oils of Leptospermum scoparium and Kunzea ericoides., Phytochemistry 50.3 (1999): 407-415]. The distillation resulted in fractions 1-3 that were enriched in some compounds compared with the unfractionated oil. Fraction 3 is enriched in the four β-triketone components leptospermone, isoleptospermone, grandiflorone and flavesone (Figure 1). The composition of the β-triketones was found to be: flavesone 15.2%; isoleptospermome 15.5%; leptospermone 68.6%, and grandiflorone 0.7%.

Manuka oil was analysed by GC/MS and gas chromatography with flame ionisation detection (GC/FID). All hardware and software were supplied by Varian Inc (Melbourne, Australia).

GC analysis of Manuka oil and its fractions was performed using a Varian 3800 gas chromatograph equipped with a flame ionisation detector (FID) and a data handling system (Varian Star Version 4). The column used was VF5-ms (30 m × 0.25 mm, 0.5 $\mu$m film thickness). The samples were dissolved in hexane (5 mg/mL). A Varian 1177 injector was used in split mode. The carrier gas was nitrogen at a flow rate of 0.4 mL/min in constant flow mode. The injection temperature was 240°C. Samples (1 $\mu$L) were injected split (20: 1). The oven temperature profile was 60°C for one min then 210°C at 6°C/min then programmed to 270°C at 25°C/min with a 5 min hold at 270°C. For GC/MS analyses, the ion source temperature was 220°C and the transfer line was held at 290°C. The range from 35 to 350 m/z was scanned three times every second. Peak areas and retention times were measured by electronic integration and quantitative composition was obtained by peak normalisation of GC/FID data. The retention indices of the compounds were also determined relative to n-alkanes.

GC/MS analysis of the samples were performed by using a Varian 3800 GC connected to a Varian 1200 triple quadrupole mass spectrometer using the same experimental conditions described above. The main components were identified by comparing both the retention indices and MS data against those of the reference standards of the NIST (National Institute of Standards and Technology) MS database and with the use of an 'in-house' library of essential oil mass spectral data accumulated over many years and through collaborative links with CSIRO (Commonwealth Scientific and Industrial Research Organization) and INRA (Institut National de la Recherche Agronomique).

### Preparing oil-loaded chitosan nanoparticles

Chitosan derived from crab shell (85% deactivation, CAS# 9012-76-4) was obtained from Sigma-Aldrich.

*Aqueous phase:* Firstly, an aqueous acetic acid solution (1% [v/v], 40 mL) was prepared and finely mixed using a magnetic stirrer at 60 ∘C for 30 min. Chitosan solution (1.5% [w/v]) was prepared by agitating chitosan in an aqueous acetic acid solution (using the above prepared solution) at ambient temperature (25-28°C). Tween 60 (0.45 g) was subsequently added to the solution (40 mL) and stirred at ambient temperature until the mixture was homogeneous [Duan, Jinghua, et al. International journal of pharmaceutics 400.1 (2010): 211-220]. pH of the solution was adjusted to 4.7-4.8 using 2N NaOH.

*Organic phase:* Soybean oil was used for preparation of the oil phase. Soybean oil (10 mL) and Span 60 (0.05 g) were mixed at 50 ∘C for 2 h and cooled to ambient temperature, then this oil phase (combined with neat Manuka oil) was gradually dropped into the aqueous chitosan solution during homogenization (~ 13,000 rpm) , under ice-bath conditions, resulting in an oil-in-water emulsion. Sodium tripolyphosphate (0.4% [w/v]) was then added drop wise into the agitated emulsion. Agitation was continuously performed for 40 min. The formed particles were collected by centrifugation and washed several times with deionised water. Finally, ultra-sonication resulted in a homogeneous suspension. The suspension/s was immediately freeze-dried.

### Preparing Manuka oil/chitosan nanoparticle formulation

The freeze-dried nanoparticles prepared above were added slowly into a pre-formulated gel base (Carbopol® 940, 1%) with continuous stirring at room temperature.

A pictorial summary of the method is illustrated in Figure 1.

### Example 1

Adults, nymphs, and nits of *P humanus capitis* were collected from a population of children between the ages of 3 and 12, with the approval of their guardians, by raking a metal louse comb through sections of the scalp. Adult and nymph lice as well as nits (eggs) were obtained and pooled by carefully removing them from the metal teeth of the comb into clean plastic boxes. The children had not been treated with any pediculicide solution for at least the preceding month, using only the louse comb.

For testing the pediculicidal activity, a filter paper diffusion bioassay was made with adults and nymphs of the insect collected from heads no more than 4 hours before carrying out the assay. After careful selection under a dissecting microscope, 10 lice in a ratio of 8:2 adult: nymph III were placed on a filter paper disk (Whatman No 1; 9-cm diameter) on the lid of a Petri dish. Next, 0.5 ml of either (a) each test solution; (b) distilled water with Tween-20 (control); and (c) distilled water, Tween-20, and preserving agents (excipient control), were spread over the lice and filter paper for each group. The commercial lotions (containing tea tree oil and permethrin as chief active components) (0.5 ml) were simultaneously run as a positive controls. Three to nine replicates were conducted for each treatment. The dishes were kept for 1 hour in a dark chamber at 26.6 ± 0.58°C and 70 6 1% humidity. At the end of this exposure period, the lice were transferred to another Petri dish with a clean filter paper disk, wet with 0.1 ml or 0.5 ml of distilled water. All the dishes were again placed in the chamber under the above-mentioned conditions and checked under a dissecting microscope 18 hours later. Lice were considered dead when they did not show any movement or peristalsis for several hours, even when they were pricked with a needle. The number of dead lice were recorded for each dish.

Ovicidal tests were carried out by placing 10 brownish oval eggs with an intact operculum on filter paper (Whatman No 1; 6-cm diameter) placed in the bottom of a Petri dish. Then, 0.25 ml of each test solution and their respective controls were applied to the nits and the paper disk. Treatment and control dishes were then incubated in a dark chamber at 29.6 ± 0.58°C with 70 6 1% humidity for 14 days, adding 0.1 ml of distilled water at 48- to 72-hour intervals in order to maintain moisture. Egg hatching was periodically monitored under a microscope, and the percentage of emergence-which includes partially hatched nits (*i.e.,* first instar nymphs that were able to lift the operculum but unable to exit)-was calculated. Each treatment was replicated three times.

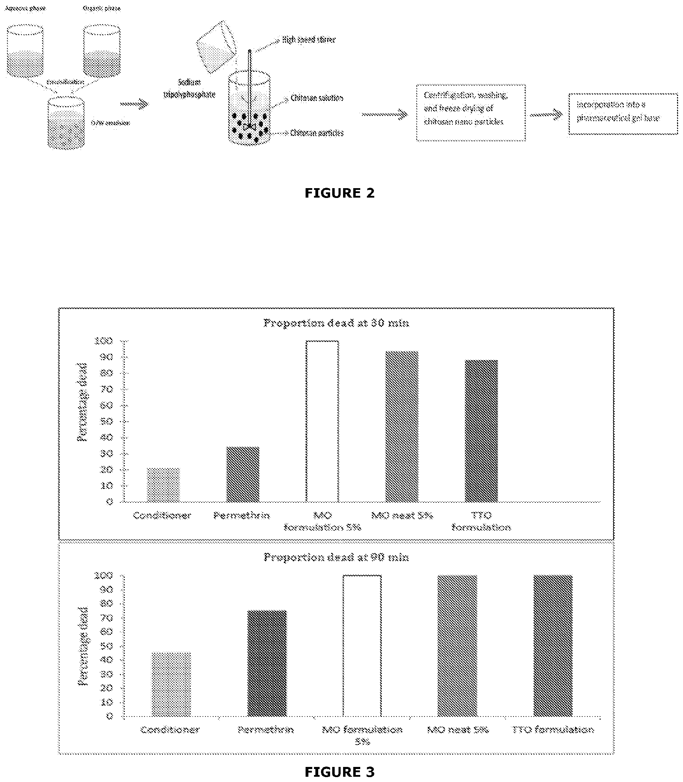

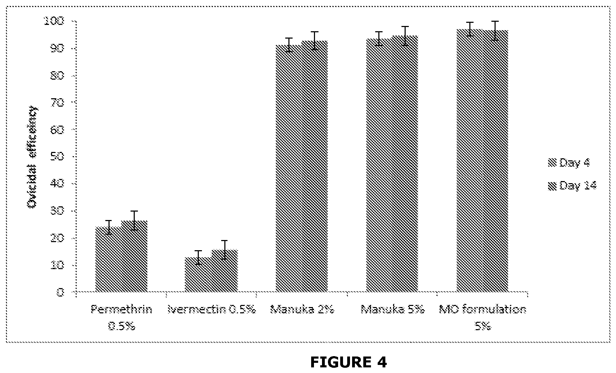

The results are depicted in Table 1 and Figures 3 and 4.

**Table 1.** Mean individual louse survival times and 95% confidence intervals when exposed to *in vitro* pediculicidal treatments, in descending order of pediculicidal activity. The MO formulation (5%) is according to the invention, whereas MO neat (5%) is comparative.

|  | Replicates | Mean survival time (min) | Std. Err. | 95% CI |
|---|---|---|---|---|
| MO formulation (5%) | 6 | 12.1 | 1.0 | 10.2-19.0 |
| MO neat (5%) | 6 | 18.1 | 1.0 | 15.2-19.0 |
| #TTO formulation | 9 | 25.8 | 3.5 | 27.8-41.7 |
| Permethrin | 4 | 56.4 | 7.9 | 41.0-71.8 |
|  | 3 |  |  | 106.8-208.2 |
| Conditioner | 7 | 157.5 | 25.9 |  |
| *Dry Control |  | 231.3 | 4.9 | 221.6-240.9 |

# Tea Tree Oil, *Filter paper

Exposure to MO (Manuka oil), both as a 5% solution in soybean oil, and nanoparticle formulation (5%, v/w), resulted in 100% mortality within 90 min, with mean survival times of 12.1 min (5% formulation) and 18.1 min (5% neat oil in soybean oil).

The mean survival time for the 5% MO nanoparticle formulation was the shortest of any of the other formulations tested and has now been shown to be extremely effective at killing lice *in vitro.* Kaplan-Meier survival analysis showed significant difference in the mortality rate of MO-nanoparticle formulation compared to other tested formulations (log rank test *P* > 0.05). Interestingly, the mean survival times of the permethrin (current gold standard, front line treatment) based formulation was substantially longer than both the MO and TTO- based formulations. Over 40% mortality was recorded in head lice exposed to plain hair conditioner after 90 minutes, a significantly lower rate (log rank test *P* < 0.001) than any of the botanical or insecticide based products.

*Example 2*

*S. scabiei* mites were isolated from scabs collected from the ears of experimentally infested pigs [Mounsey K, et al. A Tractable Experimental Model for Study of Human and Animal Scabies. Kittayapong P, ed. PLoS Neglected Tropical Diseases. 2010;4(7):e756. doi:10.1371/journal.pntd.0000756]. Scabs were placed in Petri dishes and stored in a moist chamber at 4°C overnight prior to sensitivity testing. Mite sensitivity tests were carried out essentially as previously described [Walton, Shelley F., et al. "Acaricidal activity of Melaleuca alternifolia (tea tree) oil: in vitro sensitivity of sarcoptes scabiei var hominis to tarpinen-4-ol."Archives of Dermatology 140.5 (2004): 563-566]. Neat preparations of Manuka oils and formulations were diluted to the required concentrations in the emulsifying ointment British Pharmacopoeia 88 (BP88) and cotton swabs were used to coat 35-mm plastic Petri dishes (including the lid) with one of the diluted oils, BP88 alone or benzyl benzoate 250 mg/mg. Twenty adult female mites (one per Petri dish) were tested per day. Mites were incubated in a 28°C humidified incubator and observed microscopically within the first 15-30 mins, hourly thereafter for up to 6 hours, and again at the completion of the assay (24 hrs). Mortality was recorded by microscopic verification of absence of leg movement and gut peristalsis when touched with a probe. The three fractions of Manuka oil were then diluted to the specified % in BP88 and tested as described above. Independent experiments of 20 mites per compound per day were conducted on two separate days.

To determine the ovicidal activity of Manuka oil and its fraction, dilutions to 2.5% and 5% were made in olive oil. The dilutions, olive oil alone, or ivermectin 100ug/μl in olive oil were coated onto 35mm Petri dishes, and then 20 eggs placed into each dish and the eggs observed daily for 7 days to determine the hatching rate. Six independent experiments were conducted on separate days.

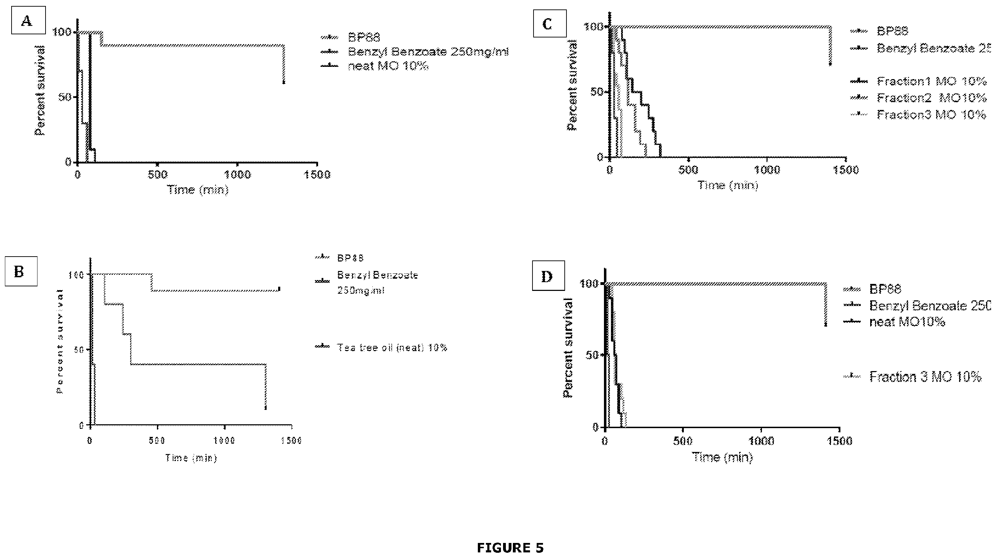

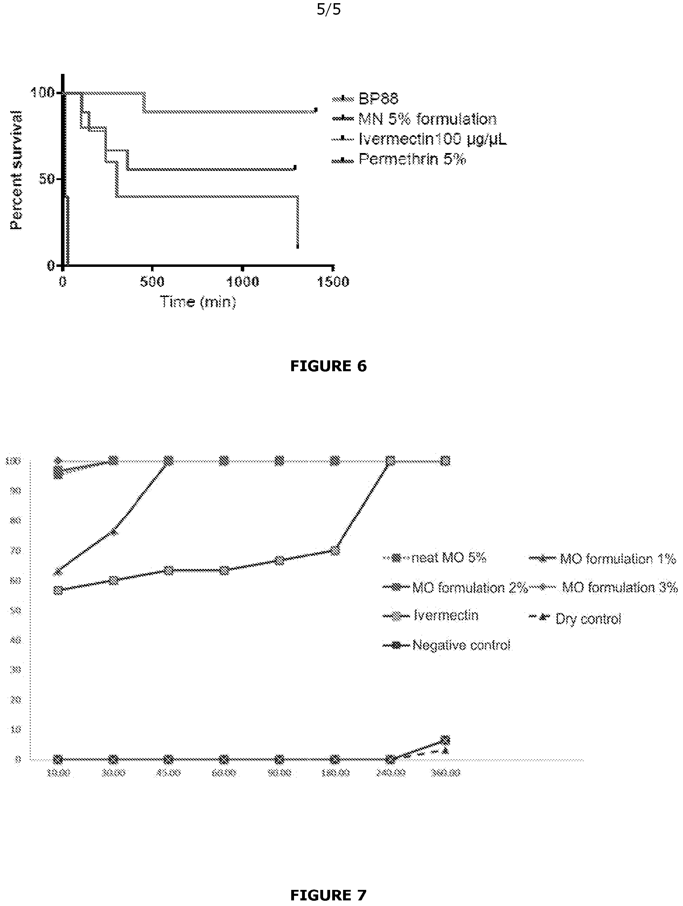

The median survival times of scabies mite populations exposed to neat Manuka oil, MO fractions, MO-nanoparticle formulation. Tea Tree Oil (TTO) and other formulations are shown in Figures 5A-D. At all concentrations tested (5%-10%), contact with MO resulted in 100% mortality of permethrin-sensitive mites after 0.41 hours, with MO-nanoparticle formulations showing remarkably higher activity compared to the current treatment standards (Figure 6).

**Table 2.** Effects of the test agents against *Sarcoptes scabiei* nits. The MO-Formulation (5%) is according to the invention, whereas the other MO treatments are comparative.

| Treatment (concentration) | Percentage inhibition | |
|---|---|---|
|  | Day 4 | Day 8 |
| Ivermectin (100μ/μL) | 12.4±5.0 (5-16) | 9.8±4.8 (5-15) |

(continued)

| Treatment (concentration) | Percentage inhibition | |
|---|---|---|
| | Day 4 | Day 8 |
| MO neat (2%) | 91.8±7.6 (82-100) | 90.8±4.6 (84-100) |
| MO fraction 3 (5%) | 96.6±2.5 (94-100) | 95.6±4.9 (88-100) |
| MO-Formulation (5%) | 98.0±1.4 (97-100) | 98.4±1.6 (96-100) |
| Negative control | 6±1.3 (4-7) | 4.8±1.3 (4-7) |

### *Ovicidal activity*

The neat MO 2% solution, fraction 3 and nanoparticle formulations showed a delay in nymph emergence (Table 2). All were significantly more effective than the control formulation of Ivermectin used in the study (Ivermectin is the current gold standard treatment for scabies management), which showed poor ovicidal activity. A similar pattern was seen against head lice nits. The MO nanoparticle formulation exerted considerably more lethal effects on parasite eggs, partially attributed to its higher permeation and viscosity, facilitating entry into the aeropyles of eggs, similar to its entry into the spiracles of skin parasites.

### *Example 3*

*Bovicola ovis* lice (used as a surrogate subspecies ectoparasite for the human louse *Pediculus humanus)* were collected from naturally infested sheep bought from rural New South Wales, Australia. Coat brushing technique was used for collection of lice from infected fleece. Lice were freshly collected on the day of establishment of each assay by trained staff and held in a temperature and humidity controlled room (i.e. 36.5 ∘C and 65% humidity) until required. The assays were carried out using methods similar to Levot, G.W. and Hughes, P.B., (Laboratory studies on resistance to cypermethrin in Damalinia ovis (Schrank)(Phthiraptera: Trichodectidae). Austral Entomology, 1990, 29(4), pp.257-259) and Callander, J.T. and James, P.J., (Insecticidal and repellent effects of tea tree (Melaleuca alternifolia) oil against Lucilia cuprina. Veterinary Parasitology, 2012, 184(2), pp.271-278) for assessing contact insecticides. Briefly, 90 mm diameter filter paper discs (Whatman No# 1) were treated with different test agents as summarised in Table 3.

**Table 3.** A brief summary of the test agents used in the assays . The MO formulations (1%, 2% and 3%) are according to the invention.

| Test products | Concentrations |
|---|---|
| MO neat (5%) | 5% v/v |
| MO formulation (1%) | 1% w/v |
| MO formulation (2%) | 2% w/v |
| MO formulation (3%) | 3% w/v |
| **Positive control** | |
| IVOMEC® (Ivermectin) | 0.8mg/mL |
| **Negative control** | |
| Dry control (filter paper) | |
| Negative control (diluent) | |

Pure Manuka oil was diluted to 5% in grape seed oil (MO neat (5%)) and the MO formulation prepared as nanoparticles as described above.

### *Treated surface assays for contact insecticides*

The stability of test, control, and/or reference agents (stored at the test conditions) was evaluated by the investigators prior to the commencement of assays. Grape seed oil was used as a diluent, and sufficient time was given to allow its full dispersion over the surface of the filter paper disc without leaving excess free fluid.

Each formulation was applied evenly onto the filter paper discs held in petri-dishes and allowed to spread/dry for approximately 1h prior to testing. After allowing time for the test agents to spread/dry, ten adult lice collected from sheep were transferred by camel's hair brush onto each paper disc and confined within a stainless steel ring (50 mm diameter, 15 mm high). Bioassays were run at 35°C and 65% relative humidity for 8 hours. The tests were carried out in triplicates with ten lice per replicate. IVOMEC® (Ivermectin 0.8mg/mL; industrial gold standard for sheep lice treatment) was used as a positive control. Two negative controls were used, a dry control using the filter paper in a petri dish with no treatment, and a control using the diluent (grape seed oil) and formulation vehicles with no active ingredients. The lice in each petri dish were closely observed for death under microscope (20X magnification) at 10 min, 30 min, 45 min, 1hr, 1.5hr, 2hr, 3hr, and 6hr time intervals as alive (active) moribund (unable to move away if touched), or dead. The number of individual dead louse was recorded at each time point. The following criteria were used for determination of death of lice. If any signs of life such as movement of antennae, gut cells or minimal legs movements are observed with stimulation by a needle, then lice are categorized as alive. The lice were judged as dead, if there are no signs of movement at all to the probe. Mortality of head lice was recorded as the mean number of dead lice across the replicate tests.

Live and dead lice were counted to determine the mortality percentages. Results of the study are expressed as a mean of mortality percentage and median survival times. Mortality in the petri dishes treated with test/positive control substances was corrected to take account of control mortality using Abbott's correction.

Corrected mortality =( [number of live control lice after treatment] - [number of live test lice after treatment ])/( [number of live control lice after treatment])*100

Classification of lousicidal effect was followed as previously described (Gemeda, N., Mokonnen, W., Lemma, H., Tadele, A., Urga, K., Addis, G., Debella, A., Getachew, M., Teka, F., Yirsaw, K. and Mudie, K., 2014. Insecticidal activity of some traditionally used Ethiopian medicinal plants against sheep ked Melophagus ovinus. Journal of parasitology research, 2014), as: Strong, mortality >80%; moderate, mortality 80-60%; weak, mortality 60-40%; little or no activity, mortality <40%.

The results from this study are summarised in Table 4 and Figure 7.

The most lousicidal products were neat (5%) MO and the 2% and 3% MO formulations. Of these high-performing products, the 3% MO formulation was the most effective, as it consistently killed 100% of lice within 10 min (0.17 hr), outperforming neat MO 5% which showed promising activity in the examples, above.

**Table 4.** Median survival times (hr) for sheep lice following exposure to different test products. The MO formulations (1%, 2% and 3%) are according to the invention.

| Test product | | Median survival time (hr) |
| --- | --- | --- |
| neat MO (5%) | | 0.50 |
| MO formulation (1%) | | 0.75 |
| MO formulation (2%) | | 0.50 |
| MO formulation (3%) | | 0.17 |
| **Positive control** | | |
| IVOMEC® (Ivermectin) | 0.8mg/mL | 4.0 |
| **Negative controls** | | |
| Untreated control* | NA | Undefined |
| Negative control** | | Undefined |

* untreated negative control - petri plates with dry filter paper and lice alone.

** negative control - petri plates treated with respective diluents or formulation vehicles (i.e., placebo formulations) with no active ingredients.

## Claims

1. An ectoparasite controlling nanoparticle composition comprising one or more β-triketones selected from leptospermone, isoleptospermone, grandiflorone and flavesone, together with a carrier, wherein the carrier is chitosan or comprises chitosan, and wherein the one or more β-triketones is a botanical extract obtained from *Leptospermum scoparium*.

2. The ectoparasite controlling nanoparticle composition according to claim 1 wherein the extract has a purity of at least 90%.

3. A nanoparticle composition as claimed in claim 1 or 2 for use in the treatment of an ectoparasitic infestation of a host subject comprising the topical administration of a nanoparticle composition according to claim 1 or 2, to said subject.

4. A non-therapeutic method for controlling an ectoparasitic infestation in a host's environment, comprising the step of applying to said environment a nanoparticle composition of claim 1 or 2.

5. The non-therapeutic method of claim 4, wherein the ectoparasitic infestation is caused by head lice, body lice, or pubic lice.

6. The non-therapeutic method of claim 4, wherein the ectoparasitic infestation is caused by a mite or a tick.

7. A non-therapeutic method of killing the eggs of an ectoparasite, comprising contacting the eggs with the nanoparticle composition according to claim 1 or 2.

8. A non-therapeutic method according to claim 7 wherein the ectoparasite is selected from the group comprising head lice, body lice or pubic lice.

9. A non-therapeutic method according to claim 7 wherein the ectoparasite is selected from a mite or a tick.

**Patentansprüche**

1. Ektoparasiten-kontrollierende Nanopartikelzusammensetzung, umfassend ein oder mehrere β-Triketone, ausgewählt aus Leptospermon, Isoleptospermon, Grandifloron und Flaveson, zusammen mit einem Träger, wobei der Träger Chitosan ist oder Chitosan umfasst und wobei das eine oder die mehreren β-Triketone ein botanischer Extrakt sind, der aus Leptospermum scoparium erhalten wurde.

2. Ektoparasiten-kontrollierende Nanopartikelzusammensetzung nach Anspruch 1, wobei der Extrakt eine Reinheit von zumindest 90 % aufweist.

3. Nanopartikelzusammensetzung nach Anspruch 1 oder 2 zur Verwendung bei der Behandlung eines Ektoparasitenbefalls eines Wirtsindividuums, umfassend die topische Verabreichung einer Nanopartikelzusammensetzung nach Anspruch 1 oder 2 an das Individuum.

4. Nichttherapeutisches Verfahren zum Kontrollieren eines Ektoparasitenbefalls in einer Umgebung eines Wirts, umfassend den Schritt des Anwendens einer Nanopartikelzusammensetzung nach Anspruch 1 oder 2 auf die Umgebung.

5. Nichttherapeutisches Verfahren nach Anspruch 4, wobei der Ektoparasitenbefall durch Kopfläuse, Körperläuse oder Filzläuse verursacht wird.

6. Nichttherapeutisches Verfahren nach Anspruch 4, wobei der Ektoparasitenbefall durch eine Milbe oder eine Zecke verursacht wird.

7. Nichttherapeutisches Verfahren zum Abtöten der Eier eines Ektoparasiten, umfassend das Kontaktieren der Eier mit einer Nanopartikelzusammensetzung nach Anspruch 1 oder 2.

8. Nichttherapeutisches Verfahren nach Anspruch 7, wobei der Ektoparasit aus der Kopfläuse, Körperläuse oder Filzläuse umfassenden Gruppe ausgewählt ist.

9. Nichttherapeutisches Verfahren nach Anspruch 7, wobei der Ektoparasit aus einer Milbe oder einer Zecke ausgewählt ist.

**Revendications**

1. Composition nanoparticulaire de lutte contre les ectoparasites comprenant une ou plusieurs ß-trichétones choisies parmi des leptospermone, isoleptospermone, grandiflorone et flavesone, conjointement avec un support, dans laquelle ledit support est du chitosane ou comprend du chitosane, et dans laquelle les une ou plusieurs ß-trichétones sont un extrait botanique obtenu à partir de *Leptospermum scoparium.*

2. Composition nanoparticulaire de lutte contre les ectoparasites selon la revendication 1, dans laquelle l'extrait botanique présente une pureté d'au moins 90 %.

3. Composition nanoparticulaire selon la revendication 1 ou 2, destinée à être utilisée dans le traitement d'une infestation ectoparasitaire d'un sujet hôte, comprenant l'administration topique audit sujet d'une composition nanoparticulaire selon la revendication 1 ou 2.

4. Procédé non thérapeutique de lutte contre une infestation ectoparasitaire dans un environnement d'hôte, comprenant l'étape consistant à appliquer audit environnement une composition nanoparticulaire selon la revendication 1 ou 2.

5. Procédé non thérapeutique selon la revendication 4, dans lequel l'infestation ectoparasitaire est causée par des poux crâniens, des poux corporels ou des poux pubiens.

6. Procédé non thérapeutique selon la revendication 4, dans lequel l'infestation ectoparasitaire est causée par un acarien ou une tique.

7. Procédé non thérapeutique de destruction des œufs d'un ectoparasite, comprenant la mise en contact des œufs avec la composition nanoparticulaire selon la revendication 1 ou 2.

8. Procédé non thérapeutique selon la revendication 7, dans lequel l'ectoparasite est choisi parmi le groupe comprenant des poux crâniens, des poux corporels ou des poux pubiens.

9. Procédé non thérapeutique selon la revendication 7, dans lequel l'ectoparasite est choisi parmi un acarien ou une tique.

**FIGURE 1**

**FIGURE 2**

**FIGURE 3**

**FIGURE 4**

FIGURE 5

EP 3 471 710 B1

**FIGURE 6**

**FIGURE 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0042982 A1

- WO 02089587 A1

**Non-patent literature cited in the description**

- *Bulletin of the World Health Organization*, February 2009, vol. 87 (2), 152
- **SPEARE** ; **RICHARD** ; **PETRA G. BUETTNER.** Head lice in pupils of a primary school in Australia and implications for control. *International journal of dermatology*, 1999, vol. 38 (4), 285-290, https://www2.health.vic.gov.au/public-health/infectious-diseases/disease-information-advice/head-lice
- **MUMCUOGLU, K.Y.** ; **MEINKING, T.A.** ; **BURKHART, C.N.** ; **BURKHART, C.G.** Head louse infestations: the "no nit" policy and its consequences. *International journal of dermatology*, 2006, vol. 45 (8), 891-896
- **CONNORS, C**. Scabies treatment.. *North. Terr. Commun. Dis. Bull.*, 1994, vol. 2, 5-6
- **THOMAS, J.** ; **PETERSON, G.M** ; **WALTON, S.F.** ; **CARSON, C.F** ; **NAUNTON, M** ; **BABY, K.E**. Scabies: an ancient global disease with a need for new therapies. *BMC infectious diseases*, 2015, vol. 15 (1), 1
- **VAN KLINK, J.W. et al.** *J. Nat. Prod.*, 1999, vol. 62 (3), 487-489
- **BRIGGS, L. H. et al.** *J Chem. Soc.*, 1948, vol. 174, 383
- **LUI, Z et al.** Polysaccharides-based nanoparticles as drug delivery systems. *Adv Drug Deliv Rev*, 2008, vol. 60 (15), 1650-1662
- **TOKUMITSU H et al.** *Chem Pharm Bull*, 1999, vol. 47, 838-842
- **El-SHABOURI MH.** *Int J Pharm*, 2002, vol. 249, 101-108
- **MITRA S et al.** *J Control Rel*, vol. 74, 317-323
- **CALVO P et al.** *J Appl Polym Sci*, 1997, vol. 63, 125-132
- **SARMENTO B et al.** *Int J Peptide Res Ther*, 2006, vol. 12, 131-138
- **TIAN XX** ; **GROVES MJ**. *J Pharm Pharmacol,*, vol. 199 (51), 151-157
- **SONGJIANG Z** ; **LIXIANG W**. *AAPS PharmsciTech*, 2009, vol. 10, 900-905
- **ANTO SM et al.** *Int J Pharm Biol Arch*, 2011, vol. 2, 926-931

- **MANCHANDA R** ; **NIMESH R**. *J Appl Polym Sci*, 2010, vol. 118, 2071-2077
- **FAN W et al.** *Colloid Surf B-Biointerfaces*, 2012, vol. 90, 21-27
- **KAIHARA S et al.** *Colloid Surf B - Biointerfaces*, 2011, vol. 85, 343-348
- **YEH MK et al.** *Acta Biomater*, 2011
- **HU Y et al.** *Biomaterials*, 2002, vol. 23, 3193-3201
- **SAJEESH S** ; **SHARMA CP**. *Int J Pharm*, 2006, vol. 325, 147-154
- **AGNIHOTRI SA** ; **AMINABHAVI TM.** *Drug Develop Ind Pharm*, 2007, vol. 33, 1254-1262
- **ATYABI F et al.** *J Nanosci Nanotechnol*, 2009, vol. 9, 4593-4603
- **GRENHA A.** Chitosan nanoparticles: a survey of preparation methods.. *Journal of drug targeting;*, 2012, vol. 20, 291-300
- Remington: The Science and Practice of Pharmacy. 2012
- **PORTER, NOEL G.** ; **ALISTAIR L. WILKINS**. Chemical, physical and antimicrobial properties of essential oils of Leptospermum scoparium and Kunzea ericoides.. *Phytochemistry*, 1999, vol. 50 (3), 407-415
- *CHEMICAL ABSTRACTS*, 9012-76-4
- **DUAN, JINGHUA et al.** *International journal of pharmaceutics*, 2010, vol. 400 (1), 211-220
- A Tractable Experimental Model for Study of Human and Animal Scabies. **MOUNSEY K et al.** PLoS Neglected Tropical Diseases. 2010, vol. 4, e756
- **WALTON, SHELLEY F et al.** Acaricidal activity of Melaleuca alternifolia (tea tree) oil: in vitro sensitivity of sarcoptes scabiei var hominis to tarpinen-4-ol. *Archives of Dermatology*, 2004, vol. 140 (5), 563-566
- **LEVOT, G.W** ; **HUGHES, P.B**. Laboratory studies on resistance to cypermethrin in Damalinia ovis (Schrank)(Phthiraptera: Trichodectidae). *Austral Entomology*, 1990, vol. 29 (4), 257-259

- **CALLANDER, J.T.** ; **JAMES, P.J.** Insecticidal and repellent effects of tea tree (Melaleuca alternifolia) oil against Lucilia cuprina. *Veterinary Parasitology*, 2012, vol. 184 (2), 271-278

- **GEMEDA, N** ; **MOKONNEN, W** ; **LEMMA, H.** ; **TADELE, A.** ; **URGA, K.** ; **ADDIS, G.** ; **DEBELLA, A.** ; **GETACHEW, M.** ; **TEKA, F.** ; **YIRSAW, K**. Insecticidal activity of some traditionally used Ethiopian medicinal plants against sheep ked Melophagus ovinus. *Journal of parasitology research*, 2014